(19) Europäisches Patentamt
European Patent Office
Office européen des brevets

(11)  **EP 2 633 457 B1**

(12)  **EUROPEAN PATENT SPECIFICATION**

(45) Date of publication and mention
of the grant of the patent:
**06.10.2021  Bulletin 2021/40**

(51) Int Cl.:
*G16H 50/30* (2018.01)      *A61B 8/04* (2006.01)
*A61B 5/0215* (2006.01)     *A61B 5/318* (2021.01)
*A61B 8/08* (2006.01)       *A61B 5/02* (2006.01)
*A61B 8/06* (2006.01)

(21) Application number: **11793651.8**

(22) Date of filing: **17.10.2011**

(86) International application number:
**PCT/EP2011/005198**

(87) International publication number:
**WO 2012/055498 (03.05.2012 Gazette 2012/18)**

(54) **METHOD FOR MYOCARDIAL SEGMENT WORK ANALYSIS**

VERFAHREN FÜR DIE MYOKARDIALE SEGMENTARBEITSANALYSE

PROCÉDÉ D'ANALYSE DU TRAVAIL DE SEGMENTS MYOCARDIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2010  US 407760 P**
**26.10.2010  EP 10188827**

(43) Date of publication of application:
**04.09.2013  Bulletin 2013/36**

(73) Proprietor: **Oslo Universitetssykehus HF**
**0450 Oslo (NO)**

(72) Inventors:
• **RUSSELL, Kristoffer**
**N-0456 Oslo (NO)**
• **SMISETH, Otto, A.**
**N-0673 Oslo (NO)**
• **ERIKSEN, Morten**
**N-0584 Oslo (NO)**

(74) Representative: **Plougmann Vingtoft a/s**
**Strandvejen 70**
**2900 Hellerup (DK)**

(56) References cited:
**US-A1- 2009 048 513**

• **STIG URHEIM ET AL: "Regional myocardial work by strain Doppler echocardiography and LV pressure: a new method for quantifying myocardial function", AMERICAN JOURNAL OF PHYSIOLOGY: HEART AND CIRCULATORY PHYSIOLOGY, AMERICAN PHYSIOLOGICAL SOCIETY, US, vol. 288, 6 January 2005 (2005-01-06), pages H2375-H2380, XP007918148, ISSN: 0363-6135, DOI: DOI::10.1152/AJPHEART.00946.2004**
• **DELGADO ET AL.: "Strain in Cardiac Resynchronization Therapy Imaging: Comparison Between Longitudinal, Circumferential, and Radial Assessment of Left Ventricular Dyssynchrony by Speckle Tracking Strain", J. AM. COLL. CARDIOL., vol. 51, 20 August 2001 (2001-08-20), pages 1944-1952, XP007918149, cited in the application**
• **CHIU W C ET AL: "Regional asynchrony of segmental contraction may explain the 'oxygen consumption paradox' in stunned myocardium", BASIC RESEARCH IN CARDIOLOGY, STEINKOPFF, DARMSTADT, DE, vol. 89, no. 2, 1 January 1994 (1994-01-01), pages 149-162, XP009146848, ISSN: 0300-8428 cited in the application**
• **PIERRE THEROUX ET AL: "Regional Myocardial Function during Acute Coronary Artery Occlision and its modification by Pharmacologic Agents in the Dog", CIRCULATION RESEARCH, GRUNE AND STRATTON, BALTIMORE, US, vol. 35, 1 December 1974 (1974-12-01), pages 896-908, XP007918150, ISSN: 0009-7330**
• **None**

**Description**

FIELD OF THE INVENTION

The present invention relates to determination of time-resolved regional mechanical power or work for individual regions or segments of a ventricle of the heart.

BACKGROUND OF THE INVENTION

Identifying patients that will respond to cardiac resynchronization therapy (CRT) has gained great interest due to the fact that 30-40% of patients selected using current guidelines does not respond or worsen after treatment with CRT. To improve this selection a clinical tool is needed that can differentiate between underlying causes of dyssynchrony.
Many echocardiographic (ECG) indices using tissue Doppler Imaging (TDI) and 2D imaging have been devised to improve selection of CRT candidates, but although initial studies were promising echocardiography has not demonstrated to improve patient selection at present.
Another important tool in clinical practise is the ability to assess regional function of the left ventricle (LV). New imaging tools such as strain either by TDI or speckle tracking have been introduced for this purpose. However, these modalities only assess regional deformation and the ability to expand assessment by for example quantifying regional work would greatly increase its clinical potential.
Delgado et al. (Strain in Cardiac Resynchronization Therapy Imaging: Comparison Between Longitudinal, Circumferential, and Radial Assessment of Left Ventricular Dyssynchrony by Speckle Tracking Strain, J. Am. Coll. Cardiol. 51 (2008): 1944-1952) describes a study where the predictive value for a positive response after CRT of radial strain (RS), circumferential strain (CS) and longitudinal strain (LS) are assessed. Indices such as maximal time delay between peak systolic strain of two segments and the standard deviation of time to peak-systolic strain were determined and evaluated.
Chiu et al. (Regional asynchrony of segmental contraction may explain the "oxygen consumption paradox" in stunned myocardium, Basic Res Cardiol. 89 (1994): 149-62) describes a study where positive contributions to myocardial work for two regions of the heart were determined as the integral of the product of force and instantaneous shortening velocity. The force and displacement were determined using force gauges and ultrasonic crystals, respectively, sutured on the myocardium. Positive work from mitral valve closure to aortic valve closure is compared to the total positive work in the whole cycle.
WO 2004/066817 describes a device for measuring pressure and dimensions of the left ventricle of the heart, and calculating among other parameters, myocardial work as the differences between an integral of the product of pressure and volume over the systole and diastole, respectively. A value for the myocardial work is thereby determined for one full cardiac cycle and for an entire heart chamber (global work).
Diamond et al. (Cardiokymography: Quantitative Analysis of Regional Ischemic Left Ventricular Dysfunction, The American Journal of Cardiol. 41 (1978): 1249-1257) describes estimating an area of a LVP (P) - relative cardiokymographic amplitude (K) loop, and reports this as an index of segment function analogous to stroke work. This index is thus determined for one full cardiac cycle.
US 2009/0048513 describes a system and method for obtaining ultrasound information relating to a heart and determining a likelihood of myocardial remodeling of the heart based on the ultrasound information by predicting a probability of myocardial remodeling and measuring local myocardial strain measurements.
STIG URHEIM ET AL: Regional myocardial work by strain Doppler echocardiography and LV pressure: a new method for quantifying myocardial function, Am J Physiol Heart Circ Physiol. 288 (2005): H2375-H2380 describes a study investigating the feasibility of quantifying regional function in terms of a segmental myocardial work index as derived from strain Doppler echocardiography (SDE) and invasive pressure.
Hence, an improved method for determining and quantifying the effects of dyssynchrony on LV function would be advantageous.

SUMMARY OF THE INVENTION

The invention relates to an apparatus as defined in claim 1 and a method as defined in claim 5. Further preferred embodiments are claimed in claims 2-4. Hence, an improved assessment of global left ventricular function, markers for regional electro-mechanical activation and function, assessment of ventricular synchronicity including classification of myocardial segments in the left ventricle, in patients with heart failure into normal, akinetic and dyskinetic segments due to late electrical activation, and assessment of viability in infarcted myocardium would be advantageous in the clinical setting. Furthermore this could directly aid patient selection in order to avoid unnecessary pacemaker implantation in the about 30 - 40 % of non-responders that are selected for CRT on basis of QRS criteria.

Thus, an object of the present invention relates to assessment of ventricular dyssynchrony in subjects that are potential candidates for cardiac resynchronisation therapy (CRT).

Calculation and presentation of segment work as a function of time based on strain imaging data (such as ultrasound strain imaging data) has not been done before. The calculations greatly improve the interpretation of combined strain and pressure recordings, and enables estimation of energetic losses caused by asynchrony of contractions. In a clinical setting this would enable the clinician to assess if the observed dyssynchrony is likely to respond to pacing.

Thus, the above described object and several other objects are intended to be obtained in the following aspects of the invention.

In a first aspect, the application provides a method for preparing and presenting data, the method comprising accessing individual strain traces for two or more myocardial segments in the left ventricle, obtained by medical imaging and a pressure trace proportional to a left ventricular pressure and in temporal synchrony with the strain traces, from a subject, wherein the strain and pressure traces are preferably obtained in a non-invasive fashion; the method further comprising the following to be carried out by an electronic processor: calculating mechanical power, P(t), and/or mechanical work, W(t), traces for the two or more individual myocardial segments in the left ventricle, from the strain traces and the pressure trace.

In an alternative formulation, the method according to the first aspect comprises calculating, on an electronic processor, mechanical power, P(t), and/or mechanical work, W(t), traces for individual myocardial segments in the left ventricle, as a function of time for a period comprising the time interval from the beginning of isovolumetric contraction and until the end of isovolumetric relaxation from ventricular tissue strain traces for individual myocardial segments in the left ventricle, and a pressure trace proportional to a left ventricular pressure and in temporal synchrony with the strain traces, strain and pressure traces preferably being obtained in a non-invasive fashion.

Compared to prior art determinations of mechanical work for individual segments using force sensors sutured to the heart described in Chiu et al., the present invention is advantageous in that it provides such determination solely from a pressure estimate and a measurement of strain, preferably by echocardiography, such as speckle tracking ultrasound imaging. This allows for a fast, easy and non-invasive determination with high temporal and spatial resolution.

Compared to work indices derived from an area of pressure - volume loops, pressure -length loops, or similar, the present invention is advantageous in that it provides such determination of power and/or work for individual myocardial segments in the left ventricle, as a function of time, and not simply the accumulated work over a full cardiac cycle. This allows for a detailed analysis of the positive and negative work contributions in the different periods of the cardiac cycle and thereby determination of more detailed indices.

In a preferred embodiment, the method further comprises simultaneously presenting the entire calculated P(t) and/or W(t) traces for the two or more myocardial segments in the left ventricle, for a period comprising the time interval from the beginning of isovolumetric contraction and until the end of isovolumetric relaxation.

In a preferred embodiment, the method according to the first aspect is a method for preparing and presenting data related to individual myocardial segment work from tissue strain imaging data, and further comprises the following to be carried out by an electronic processor: determining indices for segment work from calculated P(t) and/or W(t) traces for at least one of:

- delays between mechanical power development in myocardial segments in the left ventricle, in said time interval;
- negative work (absorption of mechanical energy) during said time interval for individual myocardial segments in the left ventricle;
- a sum of negative work for all segments as a fraction or percentage of the sum of positive or net work for all segments;
- a deviation from a standard or a mean curve;
- positive work done before and after the ejection phase (wasted work);
- wasted work, defined as positive work occurring when the aortic valve is closed;
- negative work in early systole or isovolumic contraction (IVC);
- negative work in late systole or isovolumic relaxation (IVC).

Compared to comparison of positive work from mitral valve closure to aortic valve closure to the total positive work in the whole cycle as described in Chiu et al., this embodiment is advantageous in that these indices are indicative of the effect of the contraction of an individual myocardial segment in the left ventricle, on the other segments and in that they indicate whether any such adverse effect can be reduced by CRT.

Throughout the present description the term mechanical power development is used. This term is meant to define the changes in energy, i.e. the development, measured as the mechanical energy. As a muscle contracts a certain amount of mechanical work is performed, this may be termed 'mechanical power development' Generally power is the rate at which work is performed or energy is converted. Power can also be defined as the product of a force times the velocity of its point of application, in which case work is the integral of power as a function of time. Mechanical power development in the context of this document is the rate of production of energy by contraction of myocardial tissue against forces caused by the pressure inside the chambers of the heart.

The mentioned standard or a mean curve is preferably a curve based on data from a population of patients so as to provide a statistical basis for certain values. These values could be stored in a data base or other suitable storage data structure and could e.g. be stored on a DVD/CD-ROM storage device, a hard disk or the like.

The method according to the first aspect may further comprise the initial steps of:

- accessing ventricular tissue strain traces for two or more myocardial segments in the left ventricle by medical imaging; and
- accessing a pressure trace proportional to the left ventricular pressure and in temporal synchrony with the strain traces.

In a second aspect, the application provides a computer program product for preparing data related to individual myocardial segment function in the left ventricle, the product being configured to provide the steps of the method according to the first aspect, which are to be carried out by an electronic processor. Similarly, in a third aspect, the invention provides a computer program product for updating a medical monitoring apparatus to prepare data related to individual myocardial segment function in the left ventricle, the product comprising means for installing the computer program product of the second aspect on a medical monitoring apparatus when executed.

Such computer program product for updating could be embodied by an install suite for an operating system running on a computer, or a network application for executing installation on remote computer, such as over a network connection. The computer program products of the second and third aspects may be stored and distributed on appropriate storage media such as CD ROM or a memory stick. Alternatively, they may be stored on a server and distributed via the Internet by allowing downloads of the computer program product or by running the product in a web interface.

In a fourth aspect, the application provides an apparatus for preparing and presenting data related to individual myocardial segment work in the left ventricle, from tissue strain imaging data. The apparatus may for example be implemented as or form part of a medical monitoring apparatus, a medical imaging apparatus or a medical decision support system. The apparatus comprises an electronic processor capable of calculating mechanical power, $P(t)$, and/or mechanical work, $W(t)$, traces for two or more individual myocardial segments in the left ventricle as a function of time for period comprising the time interval from the beginning of isovolumetric contraction and until the end of isovolumetric relaxation from ventricular tissue strain traces for each of the two or more myocardial segments in the left ventricle and a preferably non-invasively determined pressure trace proportional to a left ventricular pressure and in temporal synchrony with the strain traces.

The apparatus preferably further comprises a medical imaging device for recording tissue strain imaging data for two or more myocardial segments in the left ventricle. Also, the apparatus may comprise a memory in relation to the electronic processor for holding computer program products to be executed by the electronic processor. In a preferred embodiment, the apparatus is implemented by a computer connected to access tissue strain imaging data and left ventricular pressure estimate data.

In a preferred embodiment, the electronic processor is further capable of simultaneous presentation, such as on a display, of the entire calculated $P(t)$ and/or $W(t)$ traces in at least said time interval for the two or more myocardial segments.

In a preferred embodiment of the fourth aspect, the electronic processor is further capable of preparing data related to individual myocardial segment work in the left ventricle from calculated $P(t)$ and/or $W(t)$ traces for at least one of:

- delays between mechanical power development in myocardial segments in the left ventricle in said time interval;
- negative work during said time interval for individual myocardial segments in the left ventricle;
- a sum of negative work for all segments as a fraction or percentage of the sum of positive or net work for all segments;
- a deviation from a standard or a mean curve;
- wasted work, defined as positive work occurring when the aortic valve is closed;
- negative work in early systole or isovolumic contraction (IVC);
- negative work in late systole or isovolumic relaxation (IVC);

and preferably presenting determined indices.

The basic principles of the application may be applied to select subjects eligible for CRT. Thus, in a fifth aspect, the application provides a method comprising:

- calculating mechanical power, $P(t)$, and/or work, $W(t)$, traces for two or more individual segments of a subject as above; and
- selecting the subject for CRT based on a comparison of the power and/or work traces, or indices determined there from, for the segments.

In a sixth aspect, the application provides a method for assessing longitudinal changes in performance of myocardial segments in the left ventricle, the method comprising:

- determining ventricular tissue strain traces for two or more myocardial segments and a pressure trace from a subject, both the strain traces and the pressure trace being determined from the subject at least two times, at a first point in time, $T_1$, and at a second, later point in time, $T_2$;
- calculating mechanical power, P(t), and/or work, W(t), traces for each of the two or more individual segments as above for each point in time, $T_1$ and $T_2$; and
- comparing power and/or work traces, or indices derived there from, from the first and the second point in time.

It is to be understood that the present application does not provide a diagnosis as part of the work or power traces, or the indices for segment work. Rather, the invention provides information that can assist a physician, a clinician, and/or a technician in reaching a diagnosis or decide on an appropriate treatment. The use of an embodiment of the application may thus include providing information as such which is then subsequently used for diagnosing a disease or determining a treatment.

In an eight aspect the present application provides a method for preparing and presenting indices related to individual myocardial segment work in the left ventricle, the method comprising non-invasively obtaining a medical image of a segment of a heart, determining strain rate of the segment of the heart based on the medical image, determining instantaneous left ventricular pressure, and calculating segment power based on strain rate of the segment of the heart and the instantaneous left ventricular pressure. Preferably this method is computer implemented so that a processor of the computer is used for determining strain rate of the segment of the heart. The processor of the computer is preferably used for determining instantaneous left ventricular pressure for the segment of the heart. The processor of the computer is preferably used for calculating segment power based on strain rate of the segment of the heart and the instantaneous left ventricular pressure for the segment of the heart. This method is particular advantageous when dealing with images of poor quality not allowing successful speckle tracking in all segments. The method allow calculations to be performed by using strain and pressure as the only, major, inputs. The method allow calculation of work indices without having to use segment strain measurements that cover full circular cross-section of the heart. Such a simplification will allow calculation of work from any number of segments, The strain rate is expressed in $s^{-1}$ and left ventricular pressure in mmHg, and work in %mmHg.

In the following, a number of preferred and/or optional features, elements, examples, implementations and advantages will be summarized. Features or elements described in relation to one embodiment or aspect may be combined with or applied to the other embodiments or aspects where applicable. For example, structural and functional features applied in relation to a method or software implementation may also be used as features in relation to the apparatus and vice versa. Also, explanations of underlying mechanisms of the invention as realized by the inventors are presented for explanatory purposes, and should not be used in ex post facto analysis for deducing the invention.

In all aspects of the present application providing body data relating to the actual body of subject or patient being examined to e.g. a processor is likely to improve the result of the calculations. The data may include one or more of patient body weight, gender, age, body surface area to give more standardized values that may be compares across individuals. The method of correction might be determined by dimension analysis, multiple regression from data representing healthy individuals or both. Such a correction will make quantitative interpretation of work values easier.

A medical monitoring apparatus may be an apparatus capable of measuring and analysing myocardial segment strain in the left ventricle, from a subject, or apparatus capable of receiving and analysing myocardial segment strain of a subject measured by other apparatus. Typical apparatus may be MRI apparatus, CT scanners, echocardiography machines, as well as image view workstations that may or may not be coupled to any such apparatus. The medical monitoring apparatus preferably comprises a display for enabling a user to interpret or evaluate displayed data.

In the present description, with the affix of *trace* to a quantity is meant a series of values of the quantity as a function of time, so that a strain trace consists of strain values measured as a function of time, and a work trace consists of work values calculated as a function of time.

Temporal synchrony means that traces are synched in relation to temporal markers of the cardiac cycle. However, the strain and pressure traces do not need to be recorded simultaneously, but can be synched using temporal markers from a simultaneously recorded ECG, phonocardiogram, arterial peripheral blood pressure wave, plethysmogram or any other physiological variable that is known to be temporally related to the heart cycle.

In the aspects of the present invention, the parameter proportional to ventricular pressure as a function of time, p(t) and generally referred to as the pressure hereafter, may be a directly measured pressure, or may be estimated from secondary data, or may be any analogue thereto which are proportional to the left ventricular pressure (LVP). Different ways of determining p(t) will be described later. In a preferred embodiment, p(t) is measured non-invasively; several examples for such non-invasive determination of p(t) will be given.

Strain and strain rate are cardiologic imaging modalities that can be made available in real time. Strain rate measures the rate of deformation of a tissue segment measured in $s^{-1}$ and is determined by an algorithm calculating spatial differences in tissue velocities between neighbouring samples within the myocardium aligned along a direction of view. Strain is obtained by integrating strain rate over time and represents deformation of a tissue segment over time. Strain is expressed as the per cent change from the initial dimension. In the present context, the strain $s_n(t)$ is the measured strain function of segment n (of N segments) as a function of time. As mentioned, the value is typically the measured per cent strain, but may depend on the output data interface of the imaging device or its associated post-processing software, e.g. echocardiography, MRI, CTI, and others. Different ways of determining $s_n(t)$ will be described later. In a preferred embodiment, $s_n(t)$ is measured non-invasively by speckle tracking ultrasound imaging.

The power and/or work are calculated for a period comprising the time interval from the beginning of isovolumetric contraction and until the end of isovolumetric relaxation, i.e. including the entire systole. In other terms, the period preferably comprises the time interval from onset of the QRS complex in a simultaneously recorded ECG until the time point of mitral valve opening following aortic valve closure. QRS refers to the QRS complex, which is a structure on the electrocardiogram (ECG) that corresponds to the depolarization of the ventricles. Alternatively, the period preferably comprises the time interval from mitral valve closure (MVO) to mitral valve opening (MVO). In the present description, when referring to the *time interval,* the time interval as defined above is meant unless otherwise specified.

In preferred embodiments of all the above mentioned aspects, separate values of the power and/or work derived indices are calculated for the time interval only, i.e. between beginning of isovolumetric contraction (onset of the QRS complex) and the end of isovolumetric relaxation (time point of mitral valve opening), and not using power/work values from substantially outside this time interval. This is advantageous as the regional power/work in this period reveal important information so that the indices can be used as physiological markers to estimate the function of each myocardial segment in the left ventricle, as well as the potential improvement or benefit from CRT.

It is an advantage of the indices derived from the mechanical power and/or work traces in some embodiments; that they are intuitively easy to understand and interpret. This greatly improves the applicability in a clinical setting. In comparison, indices derived from modalities such as strain traces as in Delgado et al. are difficult to interpret as it is natural that the strain traces from different segments evolve differently due to their different placement and anatomy.

It follows that s(t) and p(t) values should be measured for at least the same time interval. Typically, however, $s_n(t)$ and p(t) values are measured continuously over several heartbeats. Preferably, $s_n(t)$ and p(t) are measured and values are stored in appropriate electronic memory or storage. Upon carrying out the various aspects of the invention, $s_n(t)$ and p(t) data can be retrieved from this storage by the computer. In alternative embodiments, the method is carried out by real-time processing during measurement of $s_n(t)$ and/or p(t). Here, these are preferably measured non-invasively so as not to involve surgical steps.

A work trace for the time interval typically begins and ends with a flat part and might have several increasing and decreasing parts therein between. A normal heart will have segment work traces with a monotonous rise during the time interval, without any decreasing parts. In the present description, the following terminology is relied upon:

- Negative work is defined as the accumulated absorption of mechanical energy by the current myocardial segment, i.e. energy exchanged between the segment and the surrounding tissues during elongation of the segment without respect to when in the time interval this occurs. The negative work for each segment might be calculated by integration of the segment's power trace in the time intervals where the instantaneous power has a negative value. For convenience, it might be expressed as a positive number. Equivalently, due to the relation between power and work, a value for the negative work can be determined from the work trace as the accumulated changes in the work value for all decreasing parts of the work trace within the time interval.
- Positive work is defined as the accumulated production of mechanical energy by the current myocardial segment during contraction of the segment. As for negative work, positive work might be calculated by integration of the segment's power trace in the time intervals where the instantaneous power has a positive value.Net work is defined as the net change in work value between the beginning and the end of the work trace in the time interval. With the definitions of positive and negative work above, this will be the difference between the two. For the whole myocardium, i.e. accumulated for all segments, this is approximately the same as the energy that is required to eject blood into the aortic root.
- Wasted work is defined as the positive work that does not contribute to the pumping of blood, i.e. positive work which occurs when the aortic valve is closed. This includes wasted work in IVC (before aortic valve opening), as some of the work done by early activated segments, although contributing to pressure build-up, is lost in "pushing" blood in around in the ventricle and stretching weakly and non-contracting (e.g. already contacted, late activated or ischemic) segments both before and after aortic valve closure.

The basis of the invention is calculation of temporal mechanical work (time integral of power) curves for individual myocardial segments based on recordings of local strain and an intraventricular pressure estimate. The work curves clearly discriminates myocardial segments in subjects with heart failure into normal, akinetic and dyskinetic segments due to late electrical activation.

It may be advantageous to determine the myocardial segment work for the time interval from mitral valve closure to mitral valve opening. This may easily be implemented when using a 'synthetic' waveform for the LVP determined from valvular event timing, since timing of mitral valve motion is already known. Experiments have shown that using this time period will make the method more robust against inclusion of negative work during diastolic filling, which is of increased importance for patients with elevated diastolic pressure in the left ventricle.

It may be advantageous to determine if certain segments of the heart should be excluded from the calculations. This may e.g. be for areas where part of the muscles includes necrotic or scar tissue as these areas do not contract and thus does not contribute to the work. The energy loss caused by passive distension of ischemic segments during systole will probably not be reduced by CRT treatment. Thus excluding the energy losses these segments will improve accuracy of prediction of CRT effects on overall myocardial performance.

There are numerous techniques for calculating a more accurate central (aortic root and left ventricular) pressure based on peripheral measurements, and these may be applied in combination with the present method. For example one could have used different approaches for estimating left ventricular pressure, including different mathematical methods for estimating the time course of left ventricular pressure rise during IVC and pressure fall during IVR or by using brachial artery tonometry or similar method to define timing of peak pressure and the pressure profile during the left ventricular ejection phase.

The individual aspects of the present invention may each be combined with any of the other aspects. These and other aspects of the invention will be apparent from the following description with reference to the described embodiments.

BRIEF DESCRIPTION OF THE FIGURES

Various embodiments of the invention will now be described in more detail with regard to the accompanying figures. The figures show one way of implementing the present invention and is not to be construed as being limiting to other possible embodiments falling within the scope of the attached claim set.

Figure 1 is a schematic system-chart representing an out-line of the methods or the computer program products according to embodiments of the invention.

Figures 2A and B illustrate bulls-eye plots of positive work and early/late systolic negative work in myocardial segments in a patient with left bundle branch block before (2A) and after (2B) treatment with a CRT pacemaker.

Figures 3A and B are graphs with work traces for individual segments and a mean work trace for a subject with LBBB before and after CRT, respectively.

Figures 4A and B are graphs with power traces for individual segments and a mean power trace for the same subject as in Figure 3A and B before and after CRT, respectively.

Figures 5 and 6 are graphs with work traces for a normal functioning segment and a segment with myocardial ischemia for a human subject and a dog, respectively.

Figure 7 illustrates a setup involving the medical monitoring apparatus according to an embodiment of the present invention.

Figure 8 illustrates the medical monitoring apparatus according to an embodiment of the present invention.

Figure 9 schematically illustrates a graph indicating the work of septum divided by lateral wall.

Figure 10 is a schematic scatter plot.

DETAILED DESCRIPTION OF THE INVENTION

In the following, the various aspects and embodiments of the invention will be described in more detail.

***Calculation of power and work***

The present invention applies a novel algorithm for calculating power or work for individual myocardial segments.

The algorithm is based on strain data from individual segments and pressure data common for all segments in the left ventricle, and does not require direct measurements of force.

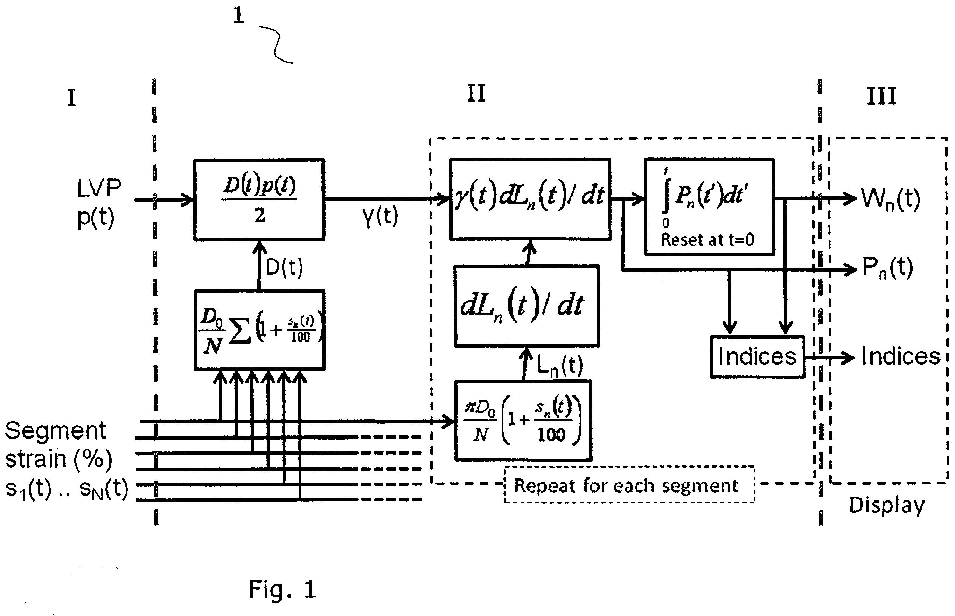

The calculation of myocardial segment mechanical power and work is done in several steps, and is preferably calculated by a computer executing an appropriate computer program. An algorithm or instruction for the calculation is illustrated in Figure 1 and will be described in detail in the following.

First, the overall changes in left ventricular diameter have to be calculated. This is done from short-axis segmental strain recordings, assuming that the segments cover the whole circumference of the left ventricle, and that they represent equal angular portions of the circumference.

The diameter of the left ventricle as a function of time (t) is calculated from segment strain measurements:

$$D(t) = \frac{D_0}{N} \sum_n \left[ 1 + \frac{s_n(t)}{100} \right], \tag{1}$$

where $D_0$ is the end-diastolic mid-wall to mid-wall diameter of the left ventricle, N is the number of equal sized myocardial segments (usually 6), and $s_n(t)$ is the measured per cent strain (elongation) of a particular segment as a function of time, the ventricular tissue strain trace.

Mechanical work by a muscle segment is ideally calculated as the temporal force times shortening velocity integral. However, due to the nature of the problem, the calculation of work has to be based on myocardial wall tension (force per unit of length) instead of force. Thus, the calculated work will also be in units of work per unit of length, which is Joule/meter. The physical term for force per unit of length is *tension,* and it is approximately calculated by using the Laplace equation for cylindrical bodies:

$$\gamma(t) = \frac{D(t)\Delta p(t)}{2}, \tag{2}$$

where $\Delta p(t)$ is the pressure difference between the inside and the outside of the left ventricle cavity. For all practical purposes, the pressure outside the cavity can be assumed to be zero (since blood pressures always are measured relative to atmospheric pressure) and the pressure inside the left ventricle or a parameter proportional to a ventricular pressure can be used as a substitute for $\Delta p(t)$, the pressure trace $p(t)$.

In order to calculate the mechanical power output from each segment, we need to know the length of the segment (L) as a function of time. This is calculated by assuming that the length of each segment is 1/N of the total circumference of the ventricle at a strain reading (s) of 0%.

$$L_n(t) = \frac{\pi D_0}{N} \left( 1 + \frac{s_n(t)}{100} \right), \tag{3}$$

Mechanical power output from a myocardial segment is calculated as the rate of change in segment length multiplied by the segment tension:

$$P_n(t) = -\gamma(t)\frac{dL_n(t)}{dt}, \tag{4}$$

where the minus sign is used since a segment shortening is associated with a positive power output.

Substituting (1), (2), and (3) into (4), the following dependency of the Power on the strain and pressure traces can be obtained:

$$P_n(t) = C_1 \cdot p(t) \cdot \frac{ds_n(t)}{dt} \cdot \sum_{n=1}^{N} \left[ 1 + \frac{s_n(t)}{100} \right], \tag{5}$$

where $C_1$ is a constant and N is the number of segments used in the segmentation of the ventricle. The diameter of the left ventricle as a function of time typically only varies approximately 15% during the systole, and may be omitted for a simpler approximate expression:

$$P_n(t) = C_1 \cdot p(t) \cdot \frac{ds_n(t)}{dt} \quad . \tag{6}$$

Finally, the work performed by each segment is found by integrating the mechanical power from each segment as a function of time:

$$W_n(t) = \int_0^t P_n(t')\,dt' + C_{2n} \quad , \tag{7}$$

where t' is an integration variable, and $C_{2n}$ is a segment specific constant chosen so that all integrals are zero at the start of systole.

The interpretation and evaluation of work traces is mainly based on their relative sizes and timing, and less on the absolute value. Hence, the proportionality constant $C_1$ may be set to 1 for simplicity.

As the above derivation shows, the regional mechanical power and work can be calculated using only measured strain traces from individual myocardial segments and a LV pressure estimate in temporal synchrony with the strain traces, both of which can be determined by non-invasive techniques. Typically, a simultaneously recorded ECG is used as a time marker to synchronize the data traces. The following describes different techniques for determining the strain and pressure traces.

### Ventricular tissue strain traces

The tissue strain imaging traces, $s_n(t)$, can be obtained non-invasively for individual myocardial segments using several different techniques.

Tissue strain imaging can be performed with ultrasound imaging, notably ultrasound tissue Doppler, speckle tracking or by recording the local thickness of the myocardium from ultrasound images or M-mode recordings (assuming that tissue is volume incompressible).

In a preferred embodiment, ultrasound speckle tracing imaging is used. Ultrasound speckle tracing imaging is based on processing of sequential greyscale ultrasound images of the myocardium, using correlation techniques to estimate the regional motion vector of the tissue. Spatial gradients in motion within a myocardial segment are used for calculation of strain.

Tissue strain imaging can also be performed with MRI, by using "tissue tagging" in a similar manner as for ultrasound speckle tracking. Tissue can be "tagged" during MRI by a spatially located pre-saturation of the tissue, typically with a line or grid pattern, using an RF-pulse with defined spectral properties in combination with a spatial magnetic field gradient. Motion and deformation (strain) of the tissue can then be followed in image sequences in a similar manner as in ultrasound speckle tracking imaging.

Preferably, all the myocardial segments together represent the entire muscle of the left ventricle. There is no common or standardized myocardial segmentation or nomenclature, rather, it is customary that the division and the number of segments depend on the imaging technology applied and the analysis one wants to make. In some applications, a segmentation based on the coronary artery territories is used. More details as well as segmentations applicable in the present invention may be found in Cerqueira et al., Standardized Myocardial Segmentation and Nomenclature for Tomographic Imaging of the Heart, American Heart Association Circulation 105 (2002), 539 and references therein.

It is understood that at least two segments are required in order to speak of segmentation. In preferred embodiments of the invention, the muscle of the left ventricle is divided into at least 4 segments, such as at least 6 segments or between 4 - 12 segments. This gives the advantage of providing a more detailed basis for evaluating the different parts of the left ventricle, without providing so many work traces that overview of and distinction between these become obsolete. Alternatively regional work done by one segment can be monitored to assess regional response to treatment or as an assessment of disease progression. As an example, one can look at one segment in the right ventricle free wall and assess work done before and after e.g. load altering or drug treatment.

### Pressure trace proportional to a ventricular pressure

The left ventricular pressure-proportional parameter, p(t), can be obtained non-invasively using several different techniques. Due to the inherent robustness of integration-based calculations (as in Equation 7), the pressure estimate does not have to be accurate. Correct timing of the recording of pressure and strain with respect to each other is however important.

In preferred embodiments, the pressure trace can be estimated non-invasively from systemic arterial pressure and pressure related temporal markers in the cardiac cycle such as cardiac valve opening and closures, apex cardiogram or phonocardiogram monitored for the subject. Alternatively, the pressure trace can be a standard pressure waveform brought into synchronization with the strain traces by non-invasively monitored, pressure related temporal markers in the cardiac cycle such as cardiac valve opening and closures. These embodiments are further explained below together with even further alternatives for determining the pressure trace.

As a surrogate for invasive pressure recordings, methods based on temporal markers in the cardiac cycle that are associated with specific pressure-related events. Such markers events are:

a) Closure of the mitral valve - start of pressure rise in systole, left ventricular pressure about 20 mmHg
b) Opening of the aortic valve - left ventricular pressure equal to diastolic systemic arterial pressure.
c) Closure of the aortic valve - left ventricular pressure slightly lower than systolic arterial pressure
d) Opening of the mitral valve - left ventricular pressure about 20 mmHg

The events listed above can be identified from ultrasound imaging of the heart, or from a phonocardiogram. A reasonable and useful estimate of the left ventricle pressure can be found by fitting a standard "average" curve to these points, such as a curve from systemic arterial pressure measurement. Alternatively, or additionally, an apex cardiogram can be used as an aid for estimating the pressure waveform shape in early systole and for determination of start and end of systolic pressure elevation within the left ventricle.

In another example, LVP can be estimated non-invasively utilizing micro bubble-based ultrasound contrast agents. Pressure dependant changes in the first, second, and subharmonic amplitudes of the ultrasound contrast agents may yield an applicable dynamic pressure estimate.

Alternatively an estimated p(t) can be determined in subjects with mitral regurgitation by estimating the velocity profile on the mitral regurgitation jet and a simplified Bernoulli equation. This may be performed on any image modality that allows estimation of the regurgitation jet velocity or similar. See e.g. Armstrong WF, Ryan T; Feigenbaum's Echocardiography; 7th ed. Lippincott Williams & Wilkins, 2009, pp 228-229 for more about this method.

In another alternative, p(t) can be estimated via linear or non-linear (power, exponential or empirical) functions. LVEDP can be estimated depending of clinical condition (non-heart failure 10, heart failure 20 mmHg), by echo measurements (E/e') or neglected (0 mmHg). The measured systolic cuff pressure (or peripheral (radial) arterial pulse wave form) can be utilized to estimate left ventricular pressure at end of IVC (LVEIVCP= lower diastolic estimated aortic pressure) as a given percentage of systolic cuff pressure. The time for EIVC can be measured by measuring aortic flow by Doppler. Depending on the function for estimated time course of LVP during IVC, the estimated start- and stop coordinates, $t_{OnsetQRS}$, LVEDP and $t_{EIVC}$, LVEIVC, can be utilized to determine the time course for LVP, and thereby a pressure trace. Measurements of aortic pressure can be done by a blood pressure device and aortic valve opening pressure can be estimated. This is then time shifted so that systolic rise coincides with aortic opening by Doppler.

Two examples of work and power traces for individual myocardial segments calculated from strain traces and pressure traces as described above are described in the following, and physiological interpretation of the work traces is presented. In these examples, left ventricular pressures have been recorded invasively by placing a catheter in the ventricle.

### Indices for segment work

In a preferred embodiment of the invention, it is preferred that indices for segment work are determined from the P(t) and/or W(t) traces. In the following, a more elaborated description of some preferred possible indices for individual myocardial segment work will be given.

### Delays between mechanical power developments in myocardial segments.

This delay may be measured by comparing a time marker in the power traces or work traces for the individual segments. As an example, the time marker $t_D$ for which 50% of the total positive work from the segment has been performed, $W_n(t_D) = 0.5 W_{n\ pos}$, may be determined for each segment. Another marker may be the time of peak power in the power trace. Yet another marker might be the center of gravity of the power curve along the time axis for each segment. In a preferred embodiment, these calculations might be restricted to be performed on the parts of the curve that has a positive power development. Other markers could also be defined. Then, having determined such a marker in each power/work trace, intersegment delays relative to a mean time, pair-wise relative delays, the standard deviation in $t_D$ or a similar parameter can be determined. A high value of the standard deviation in combination with adequate power development of individual segments means that activation of the segments is affected by dyssynchrony. A reduction in the standard deviation after treatment (CRT) indicates that the treatment was successful.

*Sum of negative work for all segments.*

The amount of negative myocardial work is an indicator of the overall energetic effectiveness of the heart, and may be estimated e.g. as the sum of negative work over all segments. It might be expressed as a fraction or percentage of the sum of positive work for all segments, such as:

$$F = \frac{W_{neg}}{W_{pos}} \quad \text{or} \quad F = 100 \cdot \frac{W_{neg}}{W_{pos}}$$

Negative work might also be expressed as a fraction of the sum of total work or net work for all segments, both of which depends on the positive work, such as:

$$F = \frac{W_{neg}}{W_{pos} - W_{neg}} \quad \text{or} \quad F = \frac{W_{neg}}{W_{net}}$$

The calculations above might be performed for any part of the heart cycle, but preferably for the time interval alone, the systole alone, or systole including the post-systolic isovolumetric relaxation phase alone. A high value of F indicates a low energetic efficacy, and a potential for improvement with CRT assuming that the myocardial segments that contribute to negative work delver a substantial net work (positive - negative) during systole and the post-systolic relaxation phase.
The net (positive minus negative) work of a segment during systole and IVR phase is the difference between the work curve at start and end of the total time interval. A robust estimate of positive work is the difference between the highest and lowest extreme values of the work curve in the same time interval, assuming that the minimum value occurs before the maximum value. The negative work might then be calculated as the difference between positive work and net work.

*Negative work (absorption of mechanical energy) for individual myocardial segments*

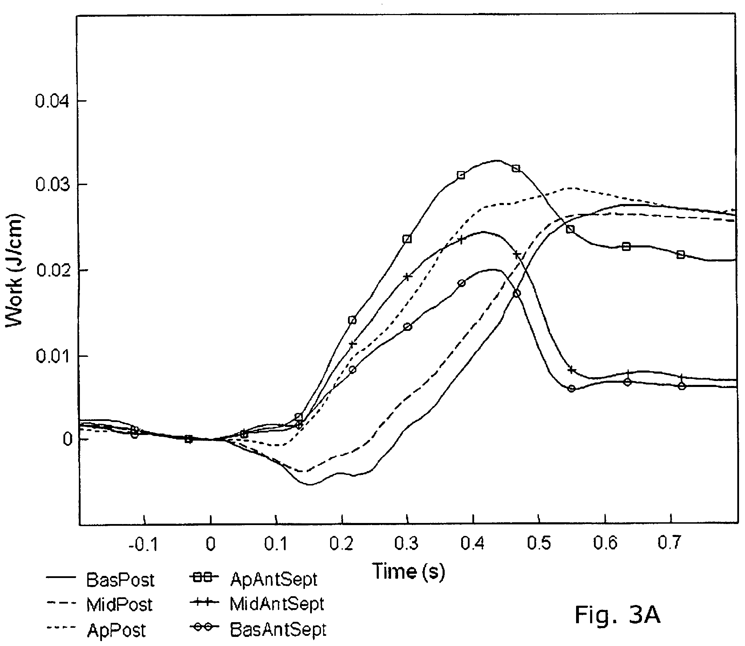

Negative work for individual myocardial segments may be expressed as a work value or alternatively as a fraction or percentage of the positive work, net work or total work as for the negative myocardial work above. A substantial value of negative work for a segment indicates that it is stretched by the contraction of another segment.
Both weak and late activated segments may show negative work in early systole. But late activated segments will respond by generating more work that continues after aortic valve closure fig 3A
Negative work occurring for a segment late in the systole or in the isovolumetric relaxation indicates that another segment is late activated so that this other segment keeps on contracting after the current segment has stopped contracting.

*Negative work for specific periods*

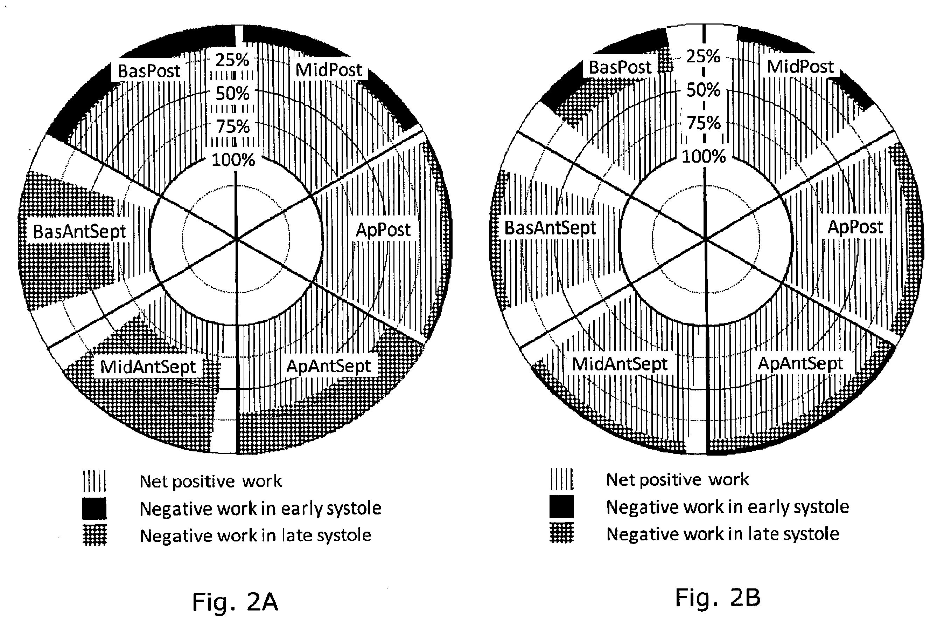

Negative work may be accumulated for specific periods of the systole, such as for the early and late systole, or similarly for the isovolumic contraction (IVC) and isovolumic relaxation (IVR). Comparison or the distribution of negative work in these periods for the different segments can provide valuable information which can subsequently be used to identify late activated or ischemic segments.
In one embodiment, illustrated in Figures 2A and B, accumulated negative work for IVC and IVR are compared with net positive work from the time interval for six LV segments. By displaying these in a "bulls-eye plots" divided into the segment positions and using different colors or shading codes for the different indices, a very intuitive reading is provided.
Figure 2A shows a bulls-eye plot of positive work and early/late systolic negative work in myocardial segments in a patient with left bundle branch block. The radial dimensions of the sectors have been scaled so that the wasted work fraction from each segment can be read directly from the concentric lines. The angular width of each sector has been scaled according to the total positive work in each segment. In this case, early systole has been defined as the time interval before left ventricular peak pressure is reached, and late systole is after the peak.

Although the physiological interpretation of these plots is still being explored, the following general "rules" are noted:

- Segments with little or no negative work are normal.
- Segments with large amounts of negative work in late systole are typically early activated segments which are stretched in the late systole
- Segments with some negative work in early systole and also some net work are typically late activated.
- Large amounts of negative work in early systole can be a marker for ischemia.

Figure 2B shows the corresponding bulls-eye plot in the patient with left bundle branch block from Figure 2A, after treatment with a CRT pacemaker implant. Note the improvements in efficacy of the septal segments.

*Wasted work for individual segments and/or for all segments*

Positive work from segments occurring within the time interval but after the time of closing of the aortic valve, such as during isovolumetric relaxation, does not contribute to the pumping of blood and is thus wasted. Wasted work for a segment or for all segments may be expressed as a work value or alternatively as a fraction or percentage of the positive work, net work or total work as for the negative myocardial work above.

Wasted work for a segment is a marker for delayed or extended contraction of the segment, typically due to late activation, or a marker of late activated segments in other parts of the heart. Depending on whether the wasted work is in IVC (the actual segment is late activated) or in IVR (other segments are late activated), wasted work accumulated for all segments indicates the potential for improved pumping function if the contributing segments are brought into synchronization. Wasted work might also occur in early activated segments before opening of the aortic valve, since energy might be absorbed by late activated or non-contracting segments.

*Deviation from a standard or a mean curve.*

Differences in the temporal development of segmental work might also be expressed by just comparing the shape of the segment work curves. One way of performing this is to calculate an average curve for all segments, and then calculate the root of mean squared sample point deviations from this curve for all segments. Since the segments of a normal heart might be expected to have differences in their power development due to geometric factors (e.g. different lengths of segments), the calculation above might also include a scaling of individual segment work curves to compensate for this.

A high value of the calculated standard deviations, either for individual segments or for all segments indicates that the heart has either a dyssynchrony in systolic contraction, or that some of the segments are unable to generate a sufficient contraction force. The latter might be caused by lack of blood supply, local myocardial disease or scarring.

Since the segments of a normal heart might be expected to have differences in their power development due to geometric factors (e.g. different lengths of segments), the calculation above might also include a scaling of individual segment work curves to compensate for this. Alternatively, in order to characterize the tissue properties instead of the length of the segments, a standard nominal length can be assumed. In yet another alternative, the work or power is expressed per unit of length squared.

A way of producing a dimensionless index that describe the similarity between each segment work curve with an arbitrary scaling and an average curve is to calculate the sample point correlation coefficient between the curves in the selected time interval. Values close to one means that the curves are very similar in their temporal development, while lower values means that the contractions are more or less asynchronous.

**Experimental protocol, selection of indices**

It is predicted that these indices all correlate with a positive response after CRT. A study in animals with surgical instrumentation for measurements of electrical activation, LVP and strain, inducing ischemia and conduction disturbances and combinations thereof will be used for identification of promising indices. A study where these indices are determined for strain trace data recorded prior to implantation of a pacemaker and then correlated with the observed response to CRT is planned. Furthermore, CRT device may turn off and on to confirm that wasted work is decreased when device is ON. This study will provide a qualification and comparison of the different indices. A further efficacy study is planned where users in a constructed clinical setting are asked to evaluate the indices on different parameters. This study will provide user response to the applicability of the different indices. Together, the results of these studies will allow for a determination of the most promising index or indices for use in a clinical setting. Furthermore, a prospective study evaluating the indices ability to predict responders to CRT might be conducted, typically using ROC analysis for each of the indices in order to identify the most promising indices and their cut-off values.

### Example 1, subject with left bundle branch block:

A subject with cardiac failure and left bundle branch block (LBBB) was imaged with ultrasound strain imaging from a short axis view, with simultaneous recording of ECG. The strain traces (as per cent elongation values) were exported together with the ECG trace as a computer file. Left ventricle pressure and ECG was recorded in a separate session and stored as a computer file.

Off-line analysis was performed by synchronizing the data from the two files by using the ECG R-wave as a marker, and segment work curves were calculated numerically as described above.

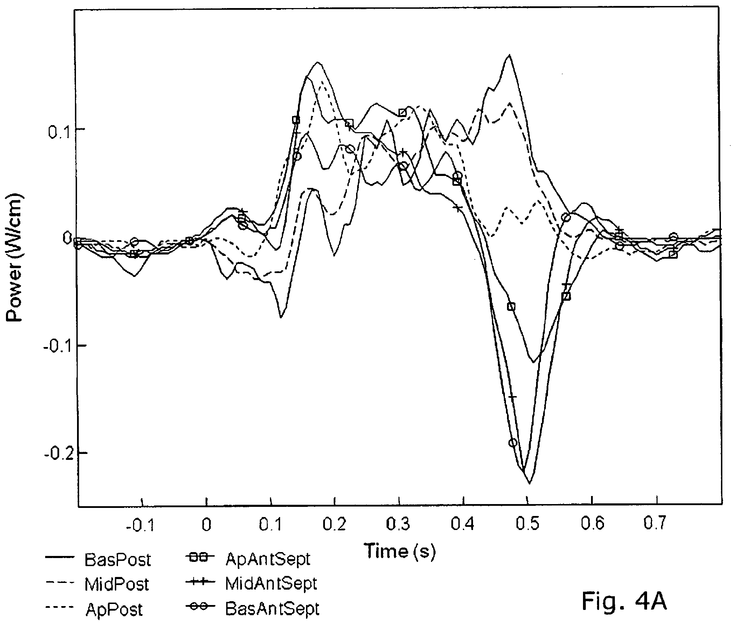

Figure 3A shows myocardial segment work in the LBBB subject, Figure 4A shows the corresponding power traces. Systole starts at about t = 0, and ends at t = 0.4 s, isovolumetric relaxation (IVR) ends at mitral valve opening, at about 0.5 s. Based on the work traces of Figure 3A, segments BasPost and MidPost show a delayed activation with negative work (stretching) early in systole, and a concomitant delay in their systolic work curves. Their contraction work continue into late systole and well into the isovolumetric relaxation, and cause other segments (ApAntSept, MidAntSept and BasAntSept) to elongate and thus absorb energy in negative work parts in the late systole and isovolumetric relaxation phases. Such negative work constitutes about 31 % of the total work in this heart. The standard deviations of the delays of power development of individual segments calculated by the centre of gravity method were 62 milliseconds, and the mean coefficient of correlation between the individual curves and the average curve of the six was 0.83.

Segments BasPost and MidPost thus performs wasted work, which represents an unwanted energetic loss, increasing myocardial $O_2$ consumption without any useful pumping activity. Despite this, the mechanical power output from these segments is considerable, strongly indicating that if they could be properly synchronized by pacemaker treatment, they would contribute normally to the pumping activity resulting in a considerable improvement in the pumping function of this heart.

### Example 2, subject with left bundle branch block and CRT pacemaker

The LBBB subject in example 1 had a cardiac resynchronization (CRT) pacemaker implanted, and the measurements and calculations in example 1 were repeated.

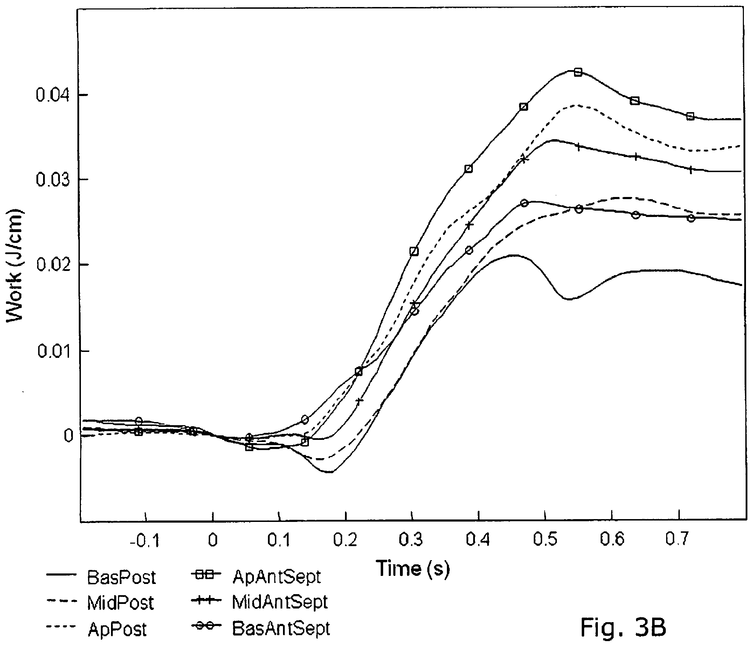

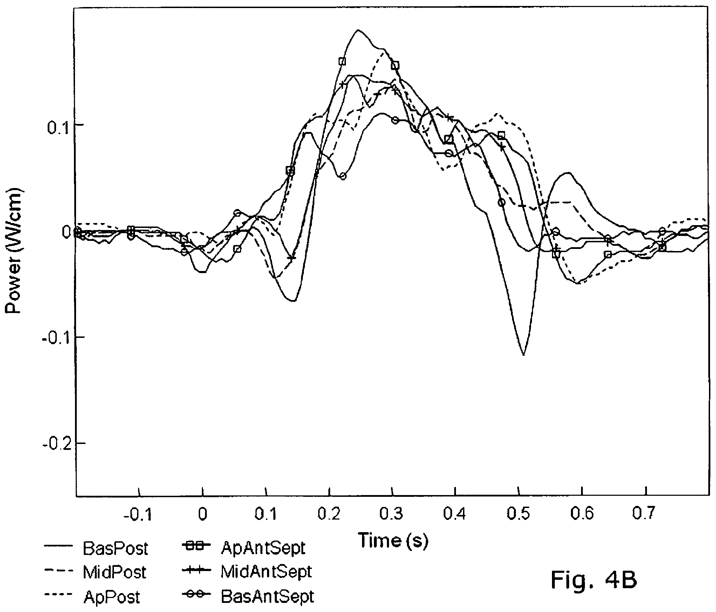

The resulting work traces are shown in Figure 3B, the corresponding power traces are shown in Figure 4B. Systole starts at about t = 0, and ends at t = 0.45, isovolumetric relaxation starts thereafter and ends at t=0.5 s. The temporal dispersion of the segment work curves has now been considerably reduced, and the total negative work has been reduced from 31 % to 11 %. In addition, the segment average net work has increased from about 0.02 J/cm (see Fig. 3A) to 0.03 J/cm. The standard deviations of the delays of power development of individual segments calculated by the centre of gravity method was reduced from 62 milliseconds to 14 milliseconds, and the mean coefficient of correlation between the individual curves and the average curve of the six was improved from 0.83 to 0.99.

### Example 3, subject with myocardial ischemia

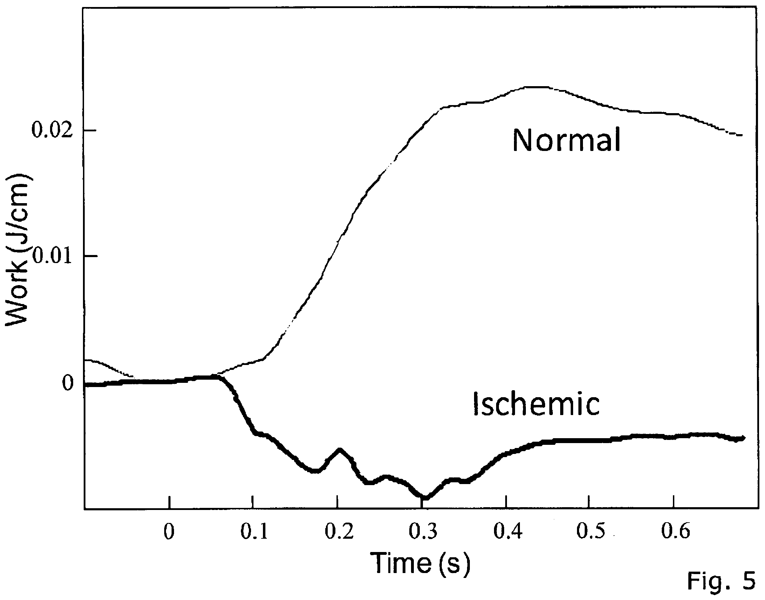

A patient with myocardial ischemia was imaged with ultrasound and the left ventricular pressure was recorded as described in example 1. Work traces were calculated for the myocardial segments. Figure 5 shows work traces for a normal segment and an ischemic segment. The systole including the IVR phase is from 0.05 to 0.45 s on the time axis. The ischemic segment absorbs mechanical energy during the pressure rise in early systole, and only a small part of this energy is delivered back by elastic recoil as the left ventricular pressure drops in late systole. This behaviour clearly discriminates between ischemic segments and segments with late electrical activation.

### Example 4, dog with myocardial ischemia.

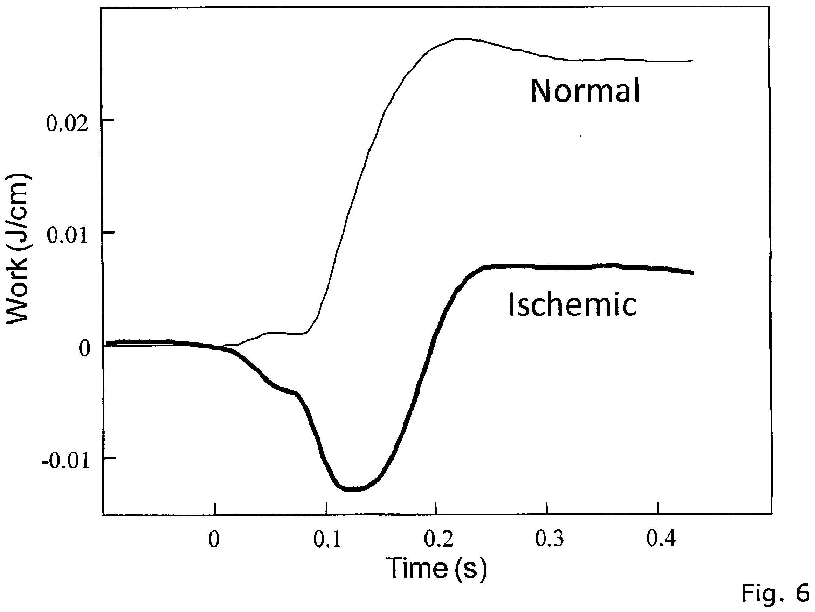

A surgically instrumented dog was studied after induction of myocardial ischemia by ligation of the left anterior descending coronary artery for 15 minutes. A simultaneous measurement of LVP and short axis ultrasound strain imaging was performed. Segment work traces for a normal and an ischemic myocardial segment were calculated and are shown in Figure 6. Systole including the isovolumetric relaxation phase last from about 0.03 to 0.27 seconds on the time axis. The ischemic segment absorbs mechanical energy in early systole, and delivers energy in late systole, probably by a combination of elastic recoil and active contraction force. The positive work contribution during systole strongly indicates that the segment still receives some blood supply, probably via collateral arteries. The total systolic work of the ischemic segment is 30% of the work provided by the normal segment, and energy loss (negative work) of the ischemic segment is 60% of the segments positive work contribution.

### *Example 5, dog with left bundle branch block*

A left bundle branch block was induced in a surgically instrumented dog by radiofrequency catheter ablation. Measurements and imaging was performed before and after the procedure as described in example 4. The standard deviations of the delays of power development of individual segments calculated by the centre of gravity method increased from 23 milliseconds to 78 milliseconds, and the mean coefficient of correlation between the individual curves and the average curve of the six was reduced from 0.98 to 0.95. The total negative work increased from 17% to 29%.

### *Method and software embodiments*

As described previously, the invention can be implemented as a method, as a computer program product (software), as software in a data analysis unit of a medical monitoring apparatus, or as software for updating a medical monitoring apparatus. In the following, embodiments of the different implementations or aspects will be described, and a detailed example of a clinical application of the invention will be described.

It should be noted that embodiments and features described in the context of one of the aspects of the present invention also apply to the other aspects of the invention.

Figure 1 represents a flow chart 1 for illustrating the architecture of an embodiment of a software product in accordance with various aspects of the invention, such as a computer program product for preparing data related to onset of active force in left ventricular muscle segments. In addition, the flow chart 1 illustrates another embodiment of the method for preparing data related to individual myocardial segment power or work in left ventricular muscle segments. Some of the steps are optional or serve to illustrate the flow of data, and are thus not part of the broadest aspects of the invention as defined by the claims.

In part I, the tissue strain $s_n(t)$ and pressure traces $p(t)$ are determined. In part II, mechanical power, $P(t)$, and/or mechanical work, $W(t)$, traces are calculated for each individual myocardial segment individually. In a preferred embodiment, indices for segment work from calculated $P(t)$ and/or $W(t)$ traces are also determined. In part III, the entire calculated $P(t)$ and/or $W(t)$ traces in the time interval are simultaneously presented for the myocardial segments. In a preferred embodiment, the determined indices are also presented.

### Applications

### *Selection of patients for CRT and setting CRT device*

Subjects have previously been selected for CRT based on observation of a prolonged QRS complex from an electrocardiogram. As mentioned previously, the basic principles of the invention may be applied to select subjects eligible for CRT based on a comparison of the calculated power and/or work traces, or indices determined there from, for the segments in accordance to the invention.

There might also be patients with normal QRS complexes (< 120 ms) that might benefit from CRT. This might be related to regional differences in the delay from electrical activation to contraction, differences in force generation or differences in the duration of force development. These can also be identified based on a comparison of the calculated power and/or work traces, or indices determined there from, for the segments in accordance to the invention.

More specific comparison criteria for the selection can comprise one or more of:

- subject is selected for CRT if a work trace for at least one segment lacks at least 20 ms, such as 30ms, 50 ms, or 70ms behind another work trace for another segment, preferably to be combined with the criteria that said at least one segment has a positive work larger than 50%, 75% or 100% of the mean net work for all segments.
- subject is selected for CRT if a sum of negative work for all segments as a fraction or percentage of the sum of positive or net work for all segments exceeds a given threshold value, wherein the given threshold value is 10% or larger, such 12% or 15%, preferably 17% or larger such as 20% or 25%.

When a subject has been selected for CRT, the calculated power and/or work traces for each left ventricular segment may be applied to improve or optimize delay settings and electrode placements for a CRT device. Today's methods rely on global measures such as LVOT flow from echocardiography (interventricular delay) and E and A wave in mitral flow (atria-ventricular delay). These measures do not give information of the regional ventricular function.

Further, for pacemaker electrode placement, these measures does not give an indication of which ventricular segment is activated the last (which is typically where the lateral electrode should be placed). To further optimise and increase the effects of using CRT devices the electrodes have to be placed at the right locations with respect to the segments. Electrode placement options are typically restricted. Currently, two electrodes are used, one at the septum and one in the lateral wall (inserted via cardiac veins). If the number of electrodes is increased, it is preferred to locate them in different segments, such as those with the longest delay in activation.

### *Longitudinal monitoring applications*

In yet another embodiment of the invention, power and/or work traces for individual segments are calculated based on strain and pressure traces determined from the subject during examinations on at least two different points in time, $T_1$ and $T_2$. Power and/or work traces, or indices derived there from, from these points in time may then be compared, and the result of the comparison may used to assess longitudinal changes in the performance of myocardial segments in the left ventricle.

Depending on the intended use, the two examinations may be performed shortly after one another, such as separated by few minutes, or with longer intervals such as several weeks or months. For some uses, a treatment of the subject or an adjustment of an existing treatment takes place between the examinations, so that the comparison can be used to observe and evaluate the effect. By repeating this, an observed effect can be used as feedback to a subsequent adjustment and the treatment can recursively be optimized. For some uses, the condition of the subject is allowed time to evolve during the examinations, so that the comparison can be used for a monitoring of the subject's development.

The following longitudinal monitoring uses are suggested:

- Monitoring the effect of CRT pacemaker treatment by comparing indices for segment work synchrony and comparing indices for "wasted work" before and after pacemaker implantation. Improved synchrony and less wasted work indicate a successful treatment.
- Optimizing the delay settings and electrode placements in CRT pacemaker treatment by observing segment work synchrony during adjustment of pacemaker delay settings and electrode placement.
- Monitoring the development and treatment of ischemic coronary arterial disease by measuring how the total work performed by each segment during systole change during the course of disease and treatment.
- Monitoring the development and treatment of myocardial disease by measuring how the total work performed by each segment during systole change during the course of disease and treatment.
- Optimizing blood pressure therapy by adjusting medication to avoid or reduce wasted work (work/power not contributing to ejection) in the left ventricle. The blood pressure medication includes all types of blood pressure medication, such as: diuretics, beta-blockers, alpha-blockers, calcium-channel blockers, angiotensin II antagonists, aldosterone antagonists, angiotensin converting enzyme antagonists, nitrates and/or any combination of the above.
- Assessing ventricular function during changes in loading conditions, in combination with blood pressure altering manoeuvres performed by or on the subject. Such manoeuvres includes hand grip test, chilling of the peripheries (hands or feet), valsalva manoeuvres, negative pressure box (lower extremities placed in negative pressure), head-up and head-down tilting, which in turn will guide blood pressure therapy as described in the previous item.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

### *Other clinical applications*

Some patients with conduction block treated by monopacing in right ventricle will over time develop remodeling and heart failure as a result of unphysiologic activation of the left ventricle causing heterogeneous work distribution. The current invention may be applied to assess work distribution after pacemaker implantation and could identify heterogeneous work distribution in this patient group. In case of heterogeneous work distribution, additional leads or replacement of the lead could be performed to avoid progression to heart failure.

### Apparatus and system embodiments

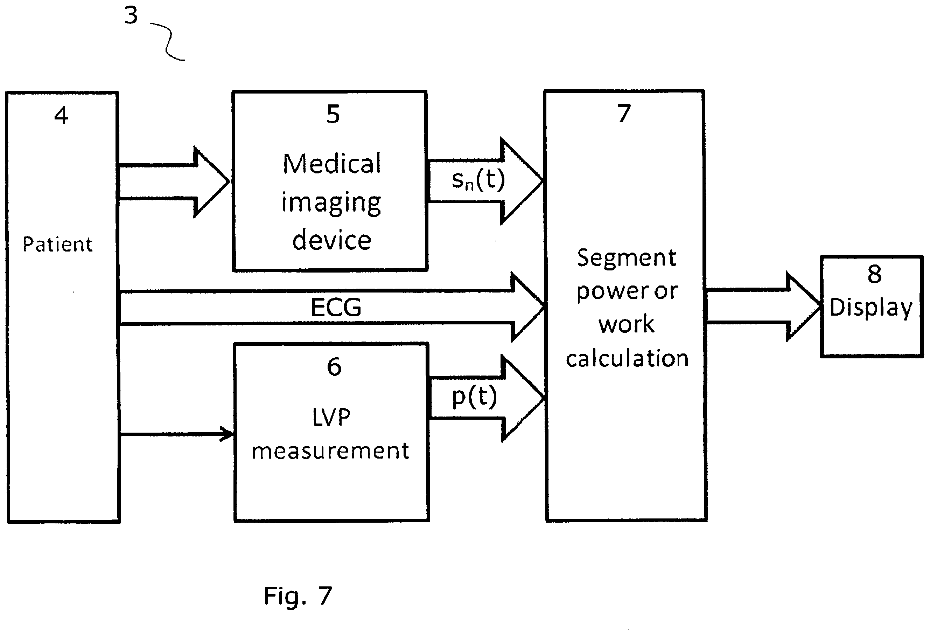

In one embodiment, the invention provides a medical monitoring apparatus or an ultrasonic or MR imaging system, both being illustrated in Figure 7.

Figure 7 illustrates the components applied in relation to the invention, and should be considered in combination with Figure 1. A medical imaging device 5, such as an ultrasonic or MR imaging apparatus, performs an imaging sequence on a subject 4 to record myocardial strain data. Different imaging modalities and techniques have been described previously. The device determines tissue strain traces for individual myocardial segments, $s_n(t)$.

Another device 6 prepares a pressure trace of a LV pressure-proportional parameter, $p(t)$. Different techniques and modalities for obtaining this have been described previously, and depending on the applied technique, the device 6 may comprise e.g. a systemic blood pressure measuring device, catheter and pressure sensor for measuring LVP invasively, ultrasound probe or phonocardiograph for determining timing events, as well as a computer for determining the pressure trace based on the measured data. There might be an overlap between devices 5 and 6.

Devices 5 and 6 thus performs the function of Part I of Figure 1, i.e. provides the strain and pressure traces.

Preferably, an electrocardiogram (ECG) is also recorded for the subject to provide a temporal reference for the heart cycle.

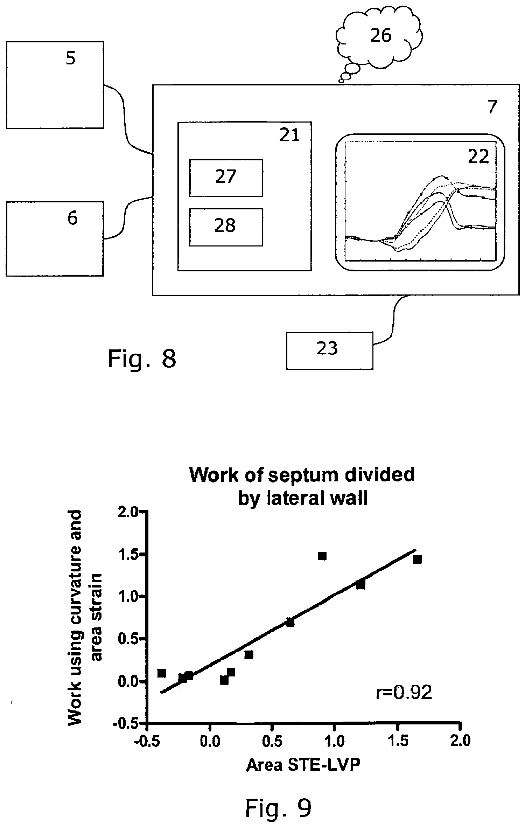

A medical monitoring apparatus 7 for preparing and presenting data is connected to devices 5 and 6 to receive the strain and pressure traces and calculate power and/or work traces according to the invention. The medical monitoring apparatus 7 comprises an electronic processor for executing computer programs and a memory for holding computer programs to be executed by the electronic processor, and preferably communication means for receiving at least measures strain data and means for presenting the calculated power/work traces and possible indices. A preferred implementation of the medical monitoring apparatus 7 is illustrated in Figure 8. Here, an embodiment of a medical monitoring apparatus 7, with a computer 21 for preparing and presenting data is shown. Computer program products for performing data preparation as described in relation to Figure 1 can be stored in memory 27 and executed by processor 28 of the computer 21. Typically, the apparatus will have a display 22 for presenting data to a user in a GUI, and a UI 23 such as a mouse and keyboard for receiving user input.

Exemplary apparatuses could be medical imaging devices such as echocardiography machines or MRI apparatuses, or work stations (such as computers) for performing analysis of data obtained from such imaging devices. Thus, the medical monitoring apparatus 7 can comprise devices 5 and/or 6 that determine $s_n(t)$ and $p(t)$, respectively. In the alternative, the medical monitoring apparatus 7 the apparatus 7 may access $s_n(t)$ and $p(t)$ data stored remotely there from, either on an external imaging device 5 or 6 or on a server or data network 26.

In both cases, the medical monitoring apparatus 7 comprise connection means to the electronic processor for receiving strain data and pressure related data to be used in the data preparation according to the invention. As mentioned above, these connection means may be to units internal or external to the medical monitoring apparatus and may comprise a data bus and/or a wired connection and/or a wireless connection.

The invention can also be embodied by a computer program product for updating a medical monitoring apparatus to prepare data related to work function in left ventricular muscle segments. Such product can be embodied as a packet managing system or an installation program for downloading and installing the software described in relation to Figure 1 on the medical monitoring apparatus 7 described in relation to Figure 7 and 8, e.g. over the network connection 26. Such program can be stored and executed by memory 27 and processor 28, or stored and executed by a server (not shown) over network connection 26.

In the embodiment where the medical monitoring apparatus 7 comprises the medical imaging devices 5 that determine $s_n(t)$, preferably in a non-invasive manner, the invention provides ultrasonic or MR imaging system operating. Such system can have a data processor corresponding to the computer 21 described above, and also involves an ultrasonic or MR imaging device configured to record ultrasound or MR data and to transmit recorded data to the data processor over the connection means.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

**Comparing work calculated from short axis strain and pressure with work calculated from area strain, curvature and pressure.**

Ideally regional myocardial work calculations should be made using area strain instead of linear strain.

There has been performed a number of studies for verifying the above discussed methods. The results are discussed in the below section.

Data from short axis and four-chamber views were used for calculation of work in the mid-septal and mid-lateral wall segments located at the intersection of these two planes. Coordinates of the mid-myocardial track points used for calculation of tangential segment strain was exported from GE Vingmed EchoPac software. These data describe the positions of about 70 points arranged along the mid-myocardial line of the left ventricle; their coordinates (x, y) are given in units of mm and are updated for each image frame. Local curvature as a function of time was found by automated fitting of a circle segment to the points located in the relevant myocardial segment, and the bi-plane average curvature ($\kappa$) expressed in units of m-1 was calculated from the segment radii from the short axis and four-chamber views:

1)  $$\kappa(t) = \frac{1}{2}\left(\frac{1}{R_{SAX}(t)} + \frac{1}{R_{4CH}(t)}\right)$$

The myocardium was approximated as a thin membrane at the mid-myocardial level. Its equivalent surface tension expressed in (N/m) was calculated from curvature and left ventricular pressure (LVP) expressed in units of Pa using the Young-Laplace equation:

2)  $$\gamma(t) = \frac{LVP(t)}{2\kappa(t)}$$

Area strain ($\varepsilon A$) was calculated from linear strain ($\varepsilon$) obtained from the two intersecting image planes:

3)  $$\varepsilon_A(t) = (1 + \varepsilon_{SAX}(t))(1 + \varepsilon_{4CH}(t)) - 1$$

Instantaneous mechanical power per unit of myocardial surface area (W/m2) was calculated from surface tension and strain rate:

4)  $$P(t) = -\gamma(t)\frac{d\varepsilon_A(t)}{dt}$$

Finally, work per unit of myocardial area (J/m2) generated during the cardiac cycle from $t_0$ to $t_1$, equivalent to the area of the surface tension - area strain loop, was calculated by integration of power:

5)  $$W = \int_{t_0}^{t} P(t)dt$$

Work calculated as described above will have physical units different from work calculated from strain and pressure loops. In order to compare data from the two methods, ratios between work generated by septal segments and work generated by lateral wall segments were calculated. Five anesthetized dogs were studied by ultrasound imaging and left ventricular pressure measurements during baseline conditions and after induction of left bundle branch block. Work from septal and lateral walls was calculated as described above, and as pressure-strain loop area derived from strain values obtained from short axis images. A scatterplot of the septum/lateral wall ratios from the two methods is shown in Fig. 9. The coefficient of correlation was 0.92

**Comparing work calculated from ultrasound imaging with myocardial glucose metabolism.**

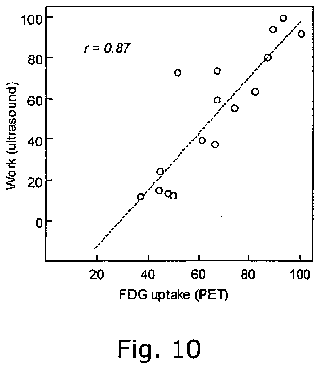

A patient with left bundle branch block and without ischemic heart disease underwent ultrasound imaging with calculation of myocardial segmental work from strain and non-invasive left ventricular pressure estimation based on valvular timing and arm cuff measurement of systolic pressure. The work values were mapped into a standard 17-segment "bulls eye" plot, and normalized to per cent values relative to the segment with the highest value. These values were compared to the corresponding values obtained from FDG-PET imaging of the myocardium according to standard clinical PET imaging procedures of myocardial glucose metabolism. The coefficient of correlation between the segmental values was 0.87, indicating that work calculated from ultrasound imaging corresponds well with tissue glucose consumption. A scatterplot of the data is shown in Fig. 10.

**References**

Chiu et al., Regional asynchrony of segmental contraction may explain the "oxygen consumption paradox" in stunned myocardium, Basic Res Cardiol. 89 (1994): 149-62.

WO 2004/066817.

Delgado et al., Strain in Cardiac Resynchronization Therapy Imaging: Comparison Between Longitudinal, Circumferential, and Radial Assessment of Left Ventricular Dyssynchrony by Speckle Tracking Strain, J. Am. Coll. Cardiol. 51 (2008): 1944-1952.

Cerqueira et al., Standardized Myocardial Segmentation and Nomenclature for Tomographic Imaging of the Heart, American Heart Association Circulation, 105 (2002): 539.

Diamond et al., Cardiokymography: Quantitative Analysis of Regional Ischemic Left Ventricular Dysfunction, The American Journal of Cardiol. 41 (1978): 1249-1257.

US 2009/0048513. STIG URHEIM ET AL: Regional myocardial work by strain Doppler echocardiography and LV pressure: a new method for quantifying myocardial function, Am J Physiol Heart Circ Physiol. 288 (2005): H2375-H2380.

## Claims

1. An apparatus for receiving, preparing and presenting data related to individual myocardial segment work from tissue strain imaging data, the apparatus comprising:

   a medical imaging device for non-invasively recording tissue strain imaging data for two or more myocardial segments in the left ventricle; and
   an electronic processor capable of:

   • calculating mechanical power, P(t), and/or mechanical work, W(t), traces for two or more individual myocardial segments in the left ventricle as a function of time for a period comprising the time interval from the beginning of isovolumetric contraction and until the end of isovolumetric relaxation from ventricular tissue strain traces for each of the two or more myocardial segments in the left ventricle and a non-invasively determined pressure trace proportional to a left ventricular pressure and in temporal synchrony with the strain traces,

   wherein the mechanical power for segment n, $P_n(t)$, is calculated from strain traces, $s_n(t)$, and the pressure trace, p(t), as:

   $$P_n(t) = C_1 \cdot p(t) \cdot \frac{ds_n(t)}{dt}$$

   or

   $$P_n(t) = C_1 \cdot p(t) \cdot \frac{ds_n(t)}{dt} \cdot \sum_{n=1}^{N} \left[ 1 + \frac{s_n(t)}{100} \right],$$

   where $C_1$ is a constant and N is the number of segments used in the segmentation of the ventricle
   and
   wherein the mechanical work for segment n, $W_n(t)$, is calculated as:

   $$W_n(t) = \int_0^t P_n(t') \, dt' + C_{2n}$$

   where t' is an integration variable and $C_{2n}$ is a constant for segment n.

2. The medical monitoring apparatus according to claim 1, wherein the electronic processor is further capable of determining the pressure trace from data representing systemic arterial pressure and pressure-related temporal markers in the cardiac cycle.

3. The medical monitoring apparatus according to claim 1, wherein the electronic processor is further capable of determining the pressure trace by bringing a standard LV-pressure waveform into synchronization with the strain traces by the use of data representing non-invasively monitored, pressure-related temporal markers in the cardiac cycle.

4. The medical monitoring apparatus according to any of the preceding claims, wherein the electronic processor is further capable of determining indices for segment work from calculated P(t) and/or W(t) traces for at least one of:

   • negative work during said time interval for individual myocardial segments in the left ventricle;
   • a sum of negative work for all segments as a fraction or percentage of the sum of positive or net work for all segments;
   • a deviation from a standard or a mean curve; and
   • wasted work, defined as positive work occurring when the aortic valve is closed.

5. A computer implemented method for preparing and presenting data related to individual myocardial segment work from tissue strain imaging data, using a computer device comprising a storage holding data relating to a standard or a mean curve for work, the method comprising:

   • non-invasively obtaining by imaging data values indicative of ventricular tissue strain traces for two or more myocardial segments in the left ventricle, and storing the data values;
   • accessing data values indicative of a pressure trace proportional to a left ventricular pressure, in temporal synchrony with the strain traces and obtained in a non-invasive fashion; and
   • using an electronic processor to calculate mechanical power, P(t), and/or mechanical work, W(t), traces for individual myocardial segments in the left ventricle as a function of time for a period comprising the time interval from the beginning of isovolumetric contraction and until the end of isovolumetric relaxation from the ventricular tissue strain traces for each of the two or more myocardial segments in the left ventricle and the pressure trace

wherein the mechanical power for segment n, $P_n(t)$, is calculated from strain traces, $s_n(t)$, and the pressure trace, $p(t)$, as:

$$P_n(t) = C_1 \cdot p(t) \cdot \frac{ds_n(t)}{dt}$$

or •

$$P_n(t) = C_1 \cdot p(t) \cdot \frac{ds_n(t)}{dt} \cdot \sum_{n=1}^{N} \left[ 1 + \frac{s_n(t)}{100} \right] ,$$

where $C_1$ is a constant and N is the number of segments used in the segmentation of the ventricle
and
wherein the mechanical work for segment n, $W_n(t)$, is calculated as:

   • ,
   •

$$W_n(t) = \int_0^t P_n(t') dt' + C_{2n}$$

where t' is an integration variable and $C_{2n}$ is a constant for segment n.

**Patentansprüche**

1. Vorrichtung zum Empfangen, Vorbereiten und Darlegen von Daten in Bezug auf die Arbeit von einzelnen Myokardsegmenten aus Gewebeverformung-Bildgebungsdaten, wobei die Vorrichtung Folgendes umfasst:

   eine medizinische Bildgebungseinrichtung zum nichtinvasiven Aufzeichnen von Gewebeverformung-Bildgebungsdaten für zwei oder mehr Myokardsegmente im linken Ventrikel; und
   einen elektronischen Prozessor, der zu Folgendem in der Lage ist:

   • Berechnen von Verläufen der mechanischen Leistung, P(t), und/oder der mechanischen Arbeit, W(t), für zwei oder mehr einzelne Myokardsegmente im linken Ventrikel in Abhängigkeit von der Zeit für einen Zeitraum beinhaltend das Zeitintervall vom Beginn der isovolumetrischen Kontraktion und bis zum Ende der isovolumetrischen Relaxation aus ventrikulären Gewebeverformung-Verläufen für jedes der zwei oder mehr Myokardsegmente im linken Ventrikel und einem nichtinvasiv bestimmten Druckverlauf proportional zu einem linksventrikulären Druck und in zeitlicher Synchronität mit den Verformung-Verläufen,

   wobei die mechanische Leistung für das Segment n, $P_n(t)$, aus Verformung-Verläufen, $s_n(t)$, und dem Druck-verlauf, $p(t)$, wie folgt berechnet wird:

   $$P_n(t) = C_1 \cdot p(t) \cdot \frac{ds_n(t)}{dt}$$

   oder

   $$P_n(t) = C_1 \cdot p(t) \cdot \frac{ds_n(t)}{dt} \cdot \sum_{n=1}^{N} \left[ 1 + \frac{s_n(t)}{100} \right],$$

   wobei $C_1$ eine Konstante ist und N die Anzahl von Segmenten ist, die in der Segmentierung des Ventrikels verwendet werden,
   und
   wobei die mechanische Arbeit für das Segment n, $W_n(t)$, wie folgt berechnet wird:

   $$W_n(t) = \int_0^t P_n(t') \, dt' + C_{2n},$$

   wobei t' eine Integrationsvariable ist und $C_{2n}$ eine Konstante für das Segment n ist.

2. Medizinische Überwachungsvorrichtung nach Anspruch 1, wobei der elektronische Prozessor ferner in der Lage ist, den Druckverlauf aus Daten, die den systemischen arteriellen Druck darstellen, und druckbezogenen zeitlichen Markern im Herzzyklus zu bestimmen.

3. Medizinische Überwachungsvorrichtung nach Anspruch 1, wobei der elektronische Prozessor ferner in der Lage ist, den Druckverlauf zu bestimmen, indem eine standardmäßige LV-Druck-Wellenform durch Verwendung von Daten, die nichtinvasiv überwachte, druckbezogene zeitliche Marker im Herzzyklus darstellen, in Synchronisation mit den Verformung-Verläufen gebracht wird.

4. Medizinische Überwachungsvorrichtung nach einem der vorstehenden Ansprüche, wobei der elektronische Prozessor ferner in der Lage ist, Indizes für Segmentarbeit aus berechneten P(t)- und/oder W(t)-Verläufen für zumindest eines der Folgenden zu bestimmen:

   • negative Arbeit während des Zeitintervalls für einzelne Myokardsegmente im linken Ventrikel;
   • eine Summe von negativer Arbeit für alle Segmente als einen Bruchteil oder einen Prozentsatz der Summe von positiver Arbeit oder Nettoarbeit für alle Segmente;
   • eine Abweichung von einer Standard- oder einer Mittelwertkurve; und

• verschwendete Arbeit, definiert als positive Arbeit, die erfolgt, wenn die Aortenklappe geschlossen ist.

**5.** Computerimplementiertes Verfahren zum Vorbereiten und Darlegen von Daten in Bezug auf die Arbeit von einzelnen Myokardsegmenten aus Gewebeverformung-Bildgebungsdaten unter Verwendung einer Computereinrichtung umfassend einen Speicher, der Daten in Bezug auf eine Standard- oder eine Mittelwertkurve für Arbeit enthält, wobei das Verfahren Folgendes umfasst:

• nichtinvasives Erhalten, durch Bildgebungsdatenwerte, die ventrikuläre Gewebeverformung-Verläufe für zwei oder mehr Myokardsegmente im linken Ventrikel angeben, und Speichern der Datenwerte;
• Zugreifen auf Datenwerte, die einen Druckverlauf proportional zu einem linksventrikulären Druck angeben, in zeitlicher Synchronität mit den Verformung-Verläufen und auf eine nichtinvasive Weise erhalten; und
• Verwenden eines elektronischen Prozessors, um Verläufe der mechanischen Leistung, P(t), und/oder der mechanischen Arbeit, W(t), für einzelne Myokardsegmente im linken Ventrikel in Abhängigkeit von der Zeit für einen Zeitraum beinhaltend das Zeitintervall vom Beginn der isovolumetrischen Kontraktion und bis zum Ende der isovolumetrischen Relaxation aus den ventrikulären Gewebeverformung-Verläufen für jedes der zwei oder mehr Myokardsegmente im linken Ventrikel und dem Druckverlauf zu berechnen,

wobei die mechanische Leistung für das Segment n, $P_n(t)$, aus Verformung-Verläufen, $s_n(t)$, und dem Druckverlauf, $p(t)$, wie folgt berechnet wird:

$$\mathbf{P_n(t)} = \ \mathbf{C_1} \cdot \mathbf{p(t)} \cdot \frac{\mathbf{ds_n(t)}}{\mathbf{dt}}$$

oder •

$$\mathbf{P_n(t)} = \ \mathbf{C_1} \cdot \mathbf{p(t)} \cdot \frac{\mathbf{ds_n(t)}}{\mathbf{dt}} \cdot \sum_{n=1}^{N} \left[ 1 + \frac{\mathbf{s_n(t)}}{\mathbf{100}} \right],$$

wobei $C_1$ eine Konstante ist und N die Anzahl von Segmenten ist, die in der Segmentierung des Ventrikels verwendet werden,
und
wobei die mechanische Arbeit für das Segment n, $W_n(t)$, wie folgt berechnet wird:

• ,
•

$$W_n(t) = \int_0^t P_n(t')\,dt' + C_{2n}$$

wobei t' eine Integrationsvariable ist und $C_{2n}$ eine Konstante für das Segment n ist.

## Revendications

**1.** Appareil permettant de recevoir, préparer et présenter des données associées à un travail de segments myocardiques individuels à partir de données d'imagerie de déformation de tissus, l'appareil comprenant :

un dispositif d'imagerie médicale destiné à enregistrer de manière non invasive des données d'imagerie de déformation de tissus pour deux segments myocardiques ou plus dans le ventricule gauche ; et
un processeur électronique pouvant :

• calculer les tracés de puissance mécanique, P(t), et/ou de travail mécanique, W(t), pour deux segments myocardiques individuels ou plus dans le ventricule gauche en fonction du temps pendant une période comprenant l'intervalle de temps depuis le début de la contraction isovolumétrique et jusqu'à la fin de la relaxation isovolumétrique à partir de tracés de déformation de tissus ventriculaires pour chacun des deux segments myocardiques ou plus dans le ventricule gauche et d'un tracé de pression déterminé de manière non invasive proportionnel à la pression ventriculaire gauche et en synchronisation temporelle avec les tracés de déformation,

la puissance mécanique pour le segment n, $P_n(t)$, étant calculée à partir des tracés de déformation, $s_n(t)$, et du tracé de pression, $p(t)$, en tant que :

$$P_n(t) = C_1 \cdot p(t) \cdot \frac{ds_n(t)}{dt}$$

ou

$$P_n(t) = C_1 \cdot p(t) \cdot \frac{ds_n(t)}{dt} \cdot \sum_{n=1}^{N} \left[ 1 + \frac{s_n(t)}{100} \right]$$

où $C_1$ représente une constante et N représente le nombre de segments utilisés dans la segmentation du ventricule
et
le travail mécanique pour le segment n, $W_n(t)$, étant calculé en tant que :
- ,

$$W_n(t) = \int_0^t P_n(t') \, dt' + C_{2n}$$

où t' représente une variable d'intégration et $C_{2n}$ représente une constante pour le segment n.

2. Appareil de surveillance médicale selon la revendication 1, ledit processeur électronique pouvant en outre déterminer le tracé de pression à partir de données représentant la pression artérielle systémique et de marqueurs temporels associés à la pression dans le cycle cardiaque.

3. Appareil de surveillance médicale selon la revendication 1, ledit processeur électronique pouvant en outre déterminer le tracé de pression en synchronisant une forme d'onde de pression VG standard avec les tracés de déformation par l'utilisation de données représentant des marqueurs temporels associés à la pression surveillés de manière non invasive dans le cycle cardiaque.

4. Appareil de surveillance médicale selon l'une quelconque des revendications précédentes, ledit processeur électronique pouvant en outre déterminer des indices pour le travail de segments à partir des tracés P(t) et/ou W(t) calculés pour au moins l'un de :

  • un travail négatif pendant ledit intervalle de temps pour des segments myocardiques individuels dans le ventricule gauche ;
  • une somme de travail négatif pour tous les segments sous la forme d'une fraction ou d'un pourcentage de la somme du travail positif ou net pour tous les segments ;
  • un écart par rapport à une référence ou à une courbe moyenne ; et
  • un travail perdu, défini comme un travail positif se produisant lorsque la valvule aortique est fermée.

5. Procédé mis en œuvre par ordinateur permettant de préparer et présenter des données associées au travail de segments myocardiques individuels à partir de données d'imagerie de déformation de tissus, à l'aide d'un dispositif informatique comprenant un stockage contenant des données associées à une référence ou à une courbe moyenne pour un travail, le procédé comprenant :

  • l'obtention de manière non invasive par imagerie de valeurs de données indiquant des tracés de déformation de tissus ventriculaires pour deux segments myocardiques ou plus dans le ventricule gauche, et le stockage des valeurs de données ;

• l'accès à des valeurs de données indiquant un tracé de pression proportionnel à la pression ventriculaire gauche, en synchronisation temporelle avec les tracés de déformation et obtenu de manière non invasive ; et

• l'utilisation d'un processeur électronique pour calculer les tracés de puissance mécanique, P(t), et/ou de travail mécanique, W(t), pour des segments myocardiques individuels dans le ventricule gauche en fonction du temps pendant une période comprenant l'intervalle de temps depuis le début de la contraction isovolumétrique et jusqu'à la fin de la relaxation isovolumétrique à partir des tracés de déformation de tissus ventriculaires pour chacun des deux segments myocardiques ou plus dans le ventricule gauche et du tracé de pression

la puissance mécanique pour le segment n, $P_n(t)$, étant calculée à partir des tracés de déformation, $s_n(t)$, et du tracé de pression, $p(t)$, en tant que :

•

$$P_n(t) = C_1 \cdot p(t) \cdot \frac{ds_n(t)}{dt}$$

ou

•

$$P_n(t) = C_1 \cdot p(t) \cdot \frac{ds_n(t)}{dt} \cdot \sum_{n=1}^{N} \left[ 1 + \frac{s_n(t)}{100} \right],$$

où $C_1$ représente une constante et N représente le nombre de segments utilisés dans la segmentation du ventricule et

le travail mécanique pour le segment n, $W_n(t)$, étant calculé en tant que :

• ,

•

$$W_n(t) = \int_0^t P_n(t') dt' + C_{2n}$$

où t' représente une variable d'intégration et $C_{2n}$ représente une constante pour le segment n.

EP 2 633 457 B1

1

I  II  III

LVP
p(t)

$$\frac{D(t)p(t)}{2}$$

γ(t)

$$\gamma(t)dL_n(t)/dt$$

$$\int_0^t P_n(t')dt'$$
Reset at t=0

$W_n(t)$

D(t)

$$\frac{D_0}{N}\sum\left(1+\frac{s_x(t)}{100}\right)$$

$$dL_n(t)/dt$$

$P_n(t)$

Segment
strain (%)
$s_1(t) .. s_N(t)$

$L_n(t)$

$$\frac{\pi D_0}{N}\left(1+\frac{s_n(t)}{100}\right)$$

Indices → Indices

Repeat for each segment

Display

Fig. 1

| IIIII | Net positive work |
| ■ | Negative work in early systole |
| ▦ | Negative work in late systole |

Fig. 2A

| IIIII | Net positive work |
| ■ | Negative work in early systole |
| ▦ | Negative work in late systole |

Fig. 2B

EP 2 633 457 B1

Fig. 3A

EP 2 633 457 B1

Fig. 3B

EP 2 633 457 B1

Fig. 4A

Fig. 4B

Fig. 5

Fig. 6

Fig. 7

Fig. 8

**Work of septum divided by lateral wall**

r=0.92

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004066817 A

- US 20090048513 A

**Non-patent literature cited in the description**

- **DELGADO et al.** Strain in Cardiac Resynchronization Therapy Imaging: Comparison Between Longitudinal, Circumferential, and Radial Assessment of Left Ventricular Dyssynchrony by Speckle Tracking Strain. *J. Am. Coll. Cardiol.,* 2008, vol. 51, 1944-1952
- **CHIU et al.** Regional asynchrony of segmental contraction may explain the "oxygen consumption paradox" in stunned myocardium. *Basic Res Cardiol.,* 1994, vol. 89, 149-62
- **DIAMOND et al.** Cardiokymography: Quantitative Analysis of Regional Ischemic Left Ventricular Dysfunction. *The American Journal of Cardiol.,* 1978, vol. 41, 1249-1257

- **STIG URHEIM et al.** Regional myocardial work by strain Doppler echocardiography and LV pressure: a new method for quantifying myocardial function. *Am J Physiol Heart Circ Physiol.,* 2005, vol. 288, H2375-H2380
- **CERQUEIRA et al.** Standardized Myocardial Segmentation and Nomenclature for Tomographic Imaging of the Heart. *American Heart Association Circulation,* 2002, vol. 105, 539
- **ARMSTRONG WF ; RYAN T.** Feigenbaum's Echocardiography. Lippincott Williams & Wilkins, 2009, 228-229
- Regional myocardial work by strain Doppler echocardiography and LV pressure: a new method for quantifying myocardial function. *Am J Physiol Heart Circ Physiol.,* 2005, vol. 288, H2375-H2380